# EUROPEAN PATENT SPECIFICATION

(11) **EP 3 840 817 B1**
(45) Date of publication and mention of the grant of the patent: **01.07.2026**
(21) Application number: 19759341.1
(22) Date of filing: 23.08.2019
(51) Int. Cl.: A61M 25/10, A61B 5/0215, A61B 5/00, A61B 17/12

(54) **A FAULT-TOLERANT ENDOVASCULAR INFLATION DEVICE**
FEHLERTOLERANTE ENDOVASKULÄRE AUFBLASVORRICHTUNG
DISPOSITIF DE GONFLAGE ENDOVASCULAIRE INSENSIBLE AUX DÉFAILLANCES

(30) Priority: 24.08.2018 DK PA201800507
(43) Date of publication of application: 30.06.2021
(73) Proprietor: Neurescue ApS, 1106 København K (DK)
(72) Inventor: FROST, Habib, 2300 København S (DK); HULDT, Olof, 21622 Limhamn (SE); NØRLØV, Mads, Bundgaard, 4000 Roskilde (DK)
(74) Representative: Inspicos P/S
(86) International application number: PCT/EP2019/072619
(87) International publication number: WO 2020/039083

(56) References cited:
- EP-A1- 2 273 921
- EP-A1- 2 273 921
- WO-A1-2009/125380
- WO-A2-2006/060688
- WO-A2-2006/060688
- WO-A2-2010/103502
- WO-A2-2010/103502
- US-A- 5 496 311
- US-A- 5 496 311
- US-A1- 2009 105 598
- US-A1- 2009 105 598
- US-A1- 2013 096 378
- US-A1- 2013 096 378
- US-A1- 2016 263 356
- US-A1- 2016 263 356

## Description

The disclosure relates an endovascular device for providing at least partial occlusion in a blood vessel in a subject. By the at least partial occlusion, the flow of blood in the blood vessel is limited or prevented. In the following, at "least partial occlusion" is for simplicity referred to simply as "occlusion". The device comprises an elongated body extending between a proximal end and a distal end. The distal end is configured by its shape and size such that it can be inserted into the blood vessel in which occlusion is desired. An inflatable member is formed about the elongated member and configured to expand upon receipt of a fluid medium by operation of an inflation means, and a sensor is arranged for sensing an occlusion parameter in the blood vessel. The disclosure further relates to a method for providing occlusion in a blood vessel. Applicant's technically related previous US 10,143,789 and WO 2017/093483.

### BACKGROUND

Cardiovascular disease contributes 30.9% of global mortality. Currently only 1 out of 10 survive a cardiac arrest to hospital discharge. It is responsible for higher mortality rates than any other disease in industrialized countries, and three-quarters of non-infectious mortality in developing countries. In the US there are around 350.000 cardiac arrests outside of hospitals; and approximately as many inside hospitals. The potential for improvement is massive.

By the early 1970s, CPR (Cardiopulmonary Resuscitation), defibrillation, and prehospital care were all in place. The introduction of automated defibrillation units (AED) expanded the possibility for prehospital treatment of cardiac arrest, and the first AED was successfully put to use by paramedics in Brighton in 1980. In spite of this, our current best practice only has the ability to achieve resuscitation, return of spontaneous circulation (ROSC), for around 25-30% of patients both in pre-hospital and in-hospital settings.

Devices and methods exist for providing occlusion in blood vessels. Such devices are used in resuscitation or in cardiac arrest to increase return of spontaneous circulation (ROSC) and expand the time window of intervention.

The existing devices require fluoroscopy guidance and the procedure of operation is complicated by the large immobile fluoroscopy machines. Accordingly, occlusion procedures are typically only carried out in controlled environments, particularly in hospitals, and only by highly trained specialist medical staff.

Medical catheters are used in a wide range of procedures, often including an inflatable member configured to provide a specific therapeutic effect or redistributing a medium. Catheterization of these inflatable members must accommodate a wide range of sizes. The inflatable member is predominantly a compliant balloon that can stretch to a wide range of final filling diameters to facilitate a thin-profiled catheter that can be used e.g. to occlude a blood vessel and which can match blood vessels in a broad range of sizes.

The size may, as an example range from 10-32 mm for e.g. aorta or vena cava occlusion. For increased safety, there is a desire for automation to prevent user error, i.e. when the device is used in an unintended way and based only on human senses and manual user interaction, there is a risk to the patient. Further, when imaging modalities, e.g. based on fluoroscopy machines, are unavailable, an increased risk may result.

Even though automatic systems in some ways may improve the safety, automatic systems may also have weaknesses. Electronics or software may fail and therefore constitute a risk to the patient or prevent completion of the procedure. For that reason, a technical prejudice is sometimes experienced against automation, and the trained medical staff tends not to rely on automation, particularly in connection with high-risk procedures.

In an emergency situation, where time is critical, use of advanced imaging capabilities is often not an option since it requires time-consuming procedures. Use of fluoroscopy, CT, or MR scanning for intravascular catheterization may therefore not be an option in an emergency situation.

It is desirable to provide a solution to the problem of safe medical intervention also when the exact diameter of the blood vessel is unknown, or when imaging is not an option. It is also desirable to allow medical intervention in an emergency situation or when a complete repertoire of devices is unavailable.

Emergency situations include among other traumatic bleedings, non-traumatic bleedings, traumatic cardiac arrest and non-traumatic cardiac arrest. However, the use of a compliant balloon blindly necessitates supraphysiological inflation pressures, defined as an arterial pressure which is higher than 129 mmHg in the 2017 AHA guidelines. This pressure is necessary for expanding the balloon material beyond the flaccid-state circumference; the balloon pressure has to counteract the physiological pressures inside the compartment and, in addition to this, stretch the compliant material beyond the flaccid-state circumference to the desired final circumference. These pressures can exceed the pressure which is desired and which is safe for the intended procedure in a blood vessel, especially in a vessel already affected by a pathological mechanism, e.g. a traumatic tear, accumulated calcification, clot, embolism or blood vessel constriction.

Excess pressure can lead to blood vessel rupture or balloon rupture when using a standard catheter. Such risks have been described in 'Resuscitative endovascular balloon occlusion of the aorta: rupture risk and implications for blind inflation by Wasicek PJ, et al. Trauma Surg Acute Care Open 2018' as well as in numerous case reports. Furthermore, the state of the art is limited in lack of feedback in being able to distinguish and verify a correct positioning and successful therapeutic intervention without advanced imaging guidance, e.g. the insertion, inflation, deflation and extraction of a catheter. These imaging techniques also currently require a multi-year specialization in addition to a medical degree to be able to correctly interpret the images which limits the availability of these procedures and leaves a residual risk to the patient in the margin for error during the interpretation. More portable techniques including ultrasound cannot be used reliable above or near bony structures, such as the chest cage and need to be interpreted as well. Even when such imaging is available, the image interpretation and manual filling and expansion of e.g. a balloon material beyond the flaccid-state circumference with a high pressure leaves a risk of errors, particularly in the hand of the user with low sensitivity and high inter-user variability. This leads to risks of tissue damage if the balloon is overinflated and has an excessive pressure and/or balloon stretch, and potential loss of therapeutic value if the harm is not correctly identified. In the known art, the pressure inside a balloon can be limited by a simple mechanical pressure relief valve or electronic pressure-limiter. This potential use as a failsafe against a supraphysiological pressure risk is unreliable with a standard compliant balloon due to the need for the valve or the limiter to be set to a supraphysiological pressure. This leads to a situation where inflation catheters are often inflated without feedback, fault-tolerance, or pressure control of where and when the inflation exercises its pressure. This increases the risk of damaging the patient's anatomy, especially in the stressful situation of an emergency condition. If only verified by human estimation, the balloon catheter could inadvertently, how rare these incidences might occur, end in e.g. an arterial branch of the aorta, in a venous vessel of the vascular system, in a dissection between layers of the aortic wall or in a tissue compartment outside of the vascular system; or raise the pressure so high at any of these locations that there is tissue damage or balloon rupture. Both tissue damage and balloon rupture may lead to serious injury to the patient.

Relevant prior-art documents are WO2009125380A1 and US5496311A.

### SUMMARY

It is an object of embodiments of the disclosure to provide an endovascular device which is attachable to the patient such that its interaction becomes independent of risk associated with user error or automation failure.

It is an object of embodiments of the disclosure to provide a device and a method by which ease-of-use, imaging-free use, and a built-in safety can mitigate for the risks involved in catheterization, including occlusion, and thereby enable methods for providing fault-tolerant and/or error-tolerant catheterization.

It is a further object to provide a device and a method by which the catheterization can be performed with low risk in near-community and hospital settings, in hands of users with minimal training, to thereby allow not only in-hospital specialists but also non-physicians and prehospital health care professionals and others to carry out such procedures.

In respect of the unmet needs in this field, the present disclosure, in a first aspect, provides an endovascular device for providing at least partial occlusion in a blood vessel in a subject. The device comprises an elongated body extending between a proximal end and a distal end. The distal end is shaped and sized to enable its insertion into the blood vessel. The device comprises an inflatable member formed about the elongated member and configured to expand upon receipt of a fluid medium by operation of an inflation means. The device further comprises a sensor for sensing an occlusion parameter in the blood vessel, the occlusion parameter being a parameter which can characterize a degree of occlusion in the blood vessel.

Devices according to the invention are defined in appended claim 1.

### LIST OF FIGURES

Figure 1 illustrates a perspective of an embodiment of said device;
Figure 2 illustrates an embodiment of said device, illustrating the connector body;
Figure 3 illustrates an embodiment of said device, illustrating a cross-sectional view of the distal end of the elongated body;
Figure 4 illustrates the embodiment of a connector body, seen from another angle, and the controller body;
Figure 5 illustrates the connector body, but without its cover in order to show the internal components in the connector body, along with the controller body without a cover. In this figure, the two bodies are connected as intended when operating the device;
Figure 6 illustrates the distal end of the elongated body; and
Figure 7 illustrates an embodiment of a connector body.

### DETAILED DESCRIPTION OF AN EMBODIMENT

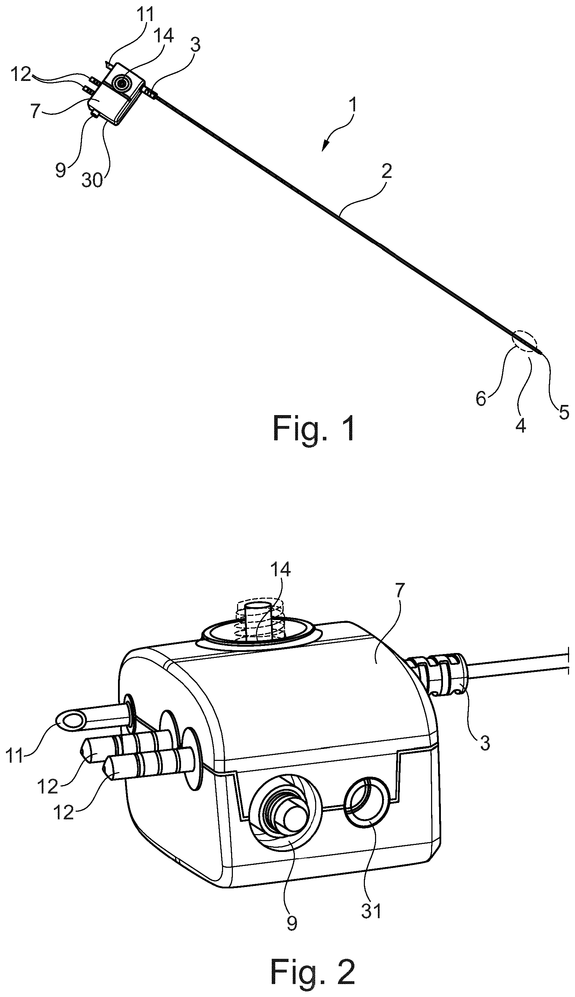

Fig. 1 illustrates the endovascular device **1.** The device comprises an elongated body **2** extending between a proximal end **3** and a distal end **4,** the distal end has a size and shape allowing its safe insertion into a blood vessel, e.g. into the aorta of a human being. For that purpose, the elongated body is terminated in a tip **5** which is made for safe insertion.

The device is configured to provide at least partly occlusion in the blood vessel by inflation of an inflatable member 6 within the blood vessel. The inflatable member **6** is formed about the elongated member and it is configured to be filled with a fluid medium and thereby expand. The fluid medium is received from an inflation means which is described in further details later. The fluid medium could be saline or a similar physiologically acceptable liquid.

The device comprises a sensor for sensing an occlusion parameter in the blood vessel. The sensor will be described in further details later, and may particularly be configured for sensing a blood pressure between the distal end and the inflatable member.

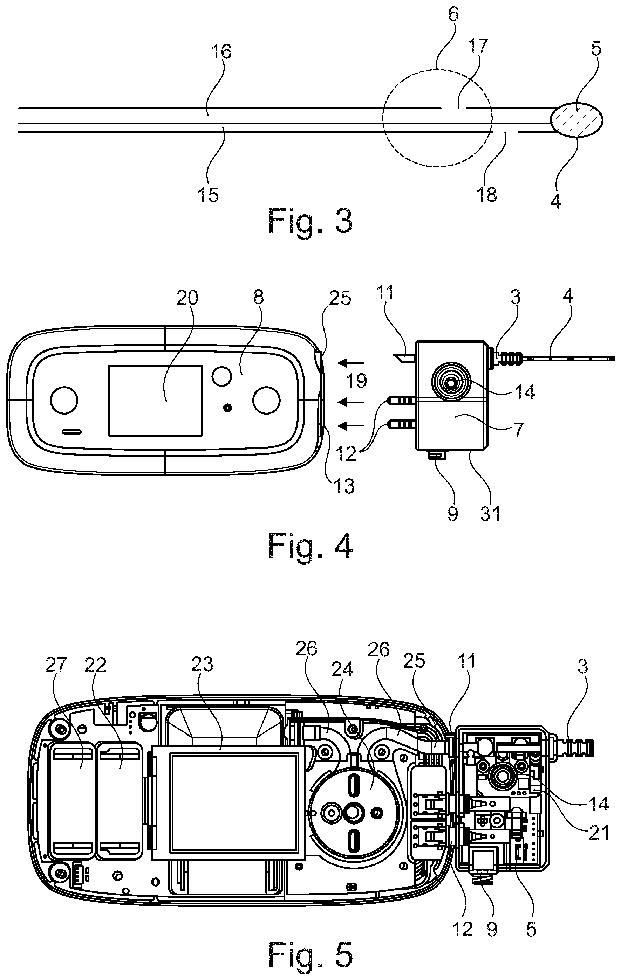

The device comprises a connector body **7** and a controller body **8.** The connector body **7** can be seen in Figs. 1 and 2, and in Figs. 4 and 5, the latter illustrating mutual functioning of the connector body **7** and the controller body **8.**

Figs. 1, 2, and 4 illustrates the connector body **7** seen from one side.

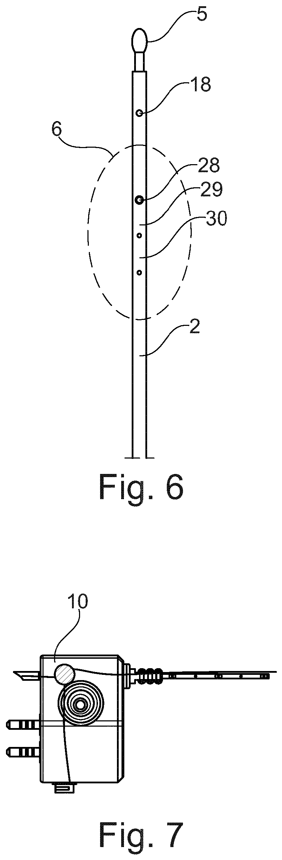

The connector body **7** comprises a first interfacing means **9,** configured to connect to a first manually operated inflation means (not shown). The first interfacing means **9** forms an external connection port and a fluid passage which connects the external connection port and a junction **10.** The junction can be seen in Fig. 7.

The connector body **7** further comprises a first fluid communication interface **11** constituting a part of the claimed second interfacing means. The first fluid communication interface forms an external connection port configured to connect to a second power controlled inflation means and a fluid connection between that external connection port and the junction **10.** The second power controlled inflation means may e.g. be a peristaltic pump, and it is located in the controller body **8.**

The connector body further forms a first electric communication interface **12** configured to electrically communicate with a second electric communication interface **13** provided in the controller body **8.**

The connector body **7** further comprises a purge structure comprising an external access port **14** configured to connect to a propagation medium container (not shown). The purge structure forms fluid connection between the connected propagation medium container and the sensor conduit **15** which is not shown in Figs. 1, 2 and 4 but which is illustrated in Fig. 3. The propagation medium container may particularly be a syringe, and the external access port **14** could be a puncture for sealing engagement with the syringe. The access port includes a valve function allowing fluid to flow from the external access port **14** to the sensor conduit **15,** but which prevents flow in the opposite direction from the sensor conduit **15** out through the external access port **14.**

The connector body **7** may be of a size making it portable, and preferably of a size making it handheld. Due to the fact that the endovascular device is supposed to be used in various environments, it is an aspect to make the connector body **7** robust to wear and tear. In an embodiment, the connector body **7** is a casing of hard plastic or any suitable material. In another embodiment, the connector body **7** is waterproof. When the connector body **7** is waterproof, the fluid connectors, i.e. the first fluid communication interface **11,** the access port **14,** and the first interfacing means **9** may be sealed, e.g. with a removable cap which prevents contamination and entrance of water or humidity into the connector body and into the conduits of the elongated body.

Fig. 3 illustrates the elongated body **2,** the inflatable member **6,** the sensor conduit **15,** and the upstream location **18.** At the upstream location, the sensor conduit forms an opening allowing pressure in the blood vessel to propagate into the sensor conduit and down to an electronic sensor converter to be discussed later.

In the illustrated embodiment, one single opening 18 is illustrated. In alternative embodiments, a plurality of openings may be provided at the upstream locations.

The inflation conduit **16** provides fluid communication between the junction **10** and thereby between the inflation means and the inflatable member. The upstream location **18** may particularly be between the distal end **4** and the inflatable member **6.**

Fig. 3 further illustrates an opening **17** between the inflation conduit **16** and the inner cavity within the inflatable member **6.**

Fig. 3 further illustrates the soft and rounded tip **5** which provides safe insertion into the blood vessel.

Fig. 4 illustrates the connector body **7** and the controller body **8.**

The connector body is attachable to the controller body by movement as indicated by the arrows **19.** The connector body is in fixed connection with the elongated body but can be released from the controller body. In that way, the connector body may be used as a stand alone product without the controller body, e.g. where automatic functions are not desired, and it can be used with the controller body where automatic functions are desired.

The controller body contains different electronic features and provides a user interface **20.**

Fig. 5 illustrates the connector body along with the controller, but exposed without an external cover.

In this view, the internal components of the connector body and the controller body are visible.

The connector body **7** comprises an electronic sensor converter **21** configured to receive a fluid signal through the sensor conduit **15.** The fluid signal represents pressure in the blood vessel above the inflatable member **6** and thereby represents an occlusion parameter.

The electronic sensor converter 21 converts the fluid signal to an electrical signal and communicates the electrical signal via the first electrical communication interface **12.**

The electrical signal transmitted via the first electrical communication interface **12** is received by the controller body **8** via the corresponding second electrical communication interface **13.**

In the controller body **8,** the electrical signal is transmitted to the electronic control unit **22** which, based on the electrical signal provides an instruction set for manual or automatic inflation.

The controller body **8** further comprises a storage body **23** containing a sufficient amount of a fluid medium for expansion of the inflatable member **6.** The fluid medium may particularly be saline or simply sterile water.

The controller body **8** further comprises a pump **24,** e.g. in the form of a peristaltic pump. The pump is connected between the storage body **23** and a fluid communication exit **25** via the pump tubing **26,** e.g. made of silicone. The fluid communication exit **25** is arranged and configured for communication with the first fluid communication interface **11** provided on the connector body **7,** the fluid communication exit 25 therefore forms a second fluid communication interface for communication with the first fluid communication interface and thereby forms part of the claimed second interfacing means, i.e. the first and second fluid communication interfaces defines the second interfacing means.

Additionally, the controller body **8** comprises a battery **27** allowing operation independent of external power.

To make the device suitable for storage over time to prevent diffusion, including diffusion of oxygen and water to and from the fluid system, one or more of the following features may be provided, since pump tubing materials are prone to diffusion, e.g. silicone tubing:
1) The material of the water bag may be configured to be diffusion resistant, e.g. by using PET/PE foil products, polypropylene, polyethylene, Polyethylene terephthalate, Polyvinyl chloride or Polyethylene laminated polyethylene terephthalate, or is made from a synthetic polymer covered with a coating or laminate of metal, hereunder including by aluminum coating.
2) The material of the pump tubing may be configured to be diffusion resistant, e.g. by using PET/PE foil products, polypropylene, polyethylene, Polyethylene terephthalate, Polyvinyl chloride or Polyethylene laminated polyethylene terephthalate, or is made from a synthetic polymer covered with a coating or laminate of metal, hereunder including by aluminum coating.
3) The fluid medium may be diffusion resistant. It may e.g. comprise or contain Xenon gas.
4) A mechanically activatable valve that opens a connection between a diffusion-resistant storage body **23** and the pump tubing **26.**
5) An electronically activatable valve that opens a connection between a diffusion-resistant storage body **23** and the pump tubing **26.**
6) Two one-sided (or a two-sided valve) between the pump tubing **26** and the storage body **23.** The one- or two-sided valve allowing the medium only to flow from the storage body 23 to the pump tubing **26** upon a pump action which draws the fluid medium into the pump tubing and pushes the medium back into the storage body upon a reverse pump action.

The connector body and/or the controller body may further include electrical components configured to measure various variables, such as temperature, sound, light, fluorescence, photoelectric effect, pressure, magnetism, flow, angular displacement, force, motion, inertia, electric impulses including ECG, EEG and EMG, glucose concentration, pO2, pCO2, SO2 or pH.

Fig. 6 illustrates three upstream locations **28, 29, 30** providing access for fluid so that various electrical components housed in the connector body **7** is capable of measuring different aspects related to the treatment.

In other embodiments, the electronic sensor converter is an analog-to-digital converter or a digital-to-analog converter and preferably a high-speed converter type, suitable for real-time data conversion and transmission. The electronic sensor converter can be controlled by means of a small computer such as a microcontroller or a microprocessor.

The internal design of the connector body **7** may prevent leakage of liquids inside the connector body **7,** and the connector body may include a liquid draining system, e.g. including a leakage connector **31.**

The connector body **7** may include its own power supply in the form of internal batteries, or it may be powered by the controller body.

Fig. 7 illustrates schematically the function of the junction **10** which forms an intersection between the first interfacing means **9** and the first fluid communication interface **11.** The junction **10** is located in the connector body **7,** and provides fluid communication from a selected one of the first interfacing means **9** and the first fluid communication interface **11** and the inflatable member such that the inflatable member can be expanded either manually via the first interfacing means or automatically via the first fluid communication interface.

### EXAMPLE 1

### Operation of a device of the disclosure

The device is inserted into the descending aorta. The device has a display and speaker.

During operation, it turns out that the device has a fault in the automatically operated inflation means which is a power controlled pump. That means that the second interfacing means are unavailable for inflation or deflation of the inflation member.

The device informs the user of the status of the device through a display and by use of a speaker. In that way, the user is instructed to inflate the inflation member via the first interfacing means. The visual and oral instructions constitute in this case at part of the instruction set for manually operated inflation. In response, the user takes a syringe and connects it to the system and manually increases the filling of the inflation member while observing an inflation parameter via the human interface of the device. The device informs the user with a GREEN symbol on the display and a confirmatory sound via the speaker once the inflation has been reached. Again, the green symbol and confirmatory sound constitutes a part of the instruction set for manually operated inflation.

Subsequently, the user decreases the filling of the balloon by way of a pre-filled syringe and the first interfacing means while observing an inflation parameter via the human interface of the device. The user is informed with a GREEN symbol on the display and a confirmatory sound via the speaker once the deflation has been reached. Again, the green symbol and confirmatory sound constitutes a part of the instruction set for manually operated inflation.

### EXAMPLE 2

### Operation of a device of the disclosure

The device is inserted into the descending aorta. The device has a display and speaker. The user operates the second interfacing means connected to a second power-controlled inflation means. This operation is carried out via an instruction set for automatically operated inflation which in this case is constituted by control codes for the power controlled pump.

The user has selected a partial inflation of the inflation member to reach a targeted inflation parameter, here in the form of a set subject blood pressure.

The device informs the user of the status of the balloon and the blood pressure via a display.

The power controlled inflation means fail due to a part fault.

The device informs the operator through the human interface and instructs the user to increase the filling of the balloon via the first interfacing means. The user instructions constitutes a part of the instruction set for manually operated inflation. The user takes a pre-filled syringe and connects it to the system and manually increases the filling of the balloon while observing the blood pressure until a desired pressure has been reached. The device informs the user with a GREEN symbol and a confirmatory sound via the speaker once the desired inflation status has been achieved. The green symbol and confirmatory sound constitutes a part of the instruction set for manually operated inflation.

Subsequently, the device informs the user that the filling should be decreased. This information is provided to the user via a RED symbol on the display and via an alarm sound through a speaker. The red symbol and confirmatory sound constitutes a part of the instruction set for manually operated inflation. In response, the user decreases the filling of the balloon by way of the pre-filled syringe and the first interfacing means while observing the blood pressure. When the intended blood pressure has been reached, the user is informed with a BLUE okay symbol on the display and a confirmatory sound from a speaker. The blue symbol and confirmatory sound constitutes a part of the instruction set for manually operated inflation.

## Claims

1. An endovascular device (1) for providing at least partial occlusion in a blood vessel in a subject, the device comprising:
- an elongated body (2) extending between a proximal end (3) and a distal end (4), the distal end being insertable into the blood vessel,
- an inflatable member (6) formed about the elongated body (2) and configured to expand upon receipt of a fluid medium from an inflation means,
- a controller body (8),
- a connector body (7) attachable to the controller body (8) and in fixed connection with the elongated body (2),
- a first interfacing means (9) comprised in the connector body (7) and forming an external connection port configured to connect to a first manually operated inflation means,
- a junction (10) housed in the connector body and connected to the external connection port by a fluid passage,
- an inflation conduit (16) providing fluid communication between the inflatable member and the junction (10),
- a first fluid communication interface (11) comprised in the connector body (7),
- a first electric communication interface (12) comprised in the connector body (7),
- a second power controlled inflation means located in the controller body (8),
- a second fluid communication interface (25) provided in the controller body (8), the first fluid communication interface (11) and the second fluid communication interface (25) configured to connect to the second power controlled inflation means and being in fluid communication with the junction (10),
- an electronic control unit (22) configured to receive an electrical signal representing an occlusion parameter and to provide an instruction set for manually operated inflation or for automatically operated inflation based on the occlusion parameter,
- a second electric communication interface (13) provided in the controller body (8) and configured to electrically communicate with the first electric communication interface (12), and
- an electronic sensor converter (21) contained in the connector body (7) and configured to receive a fluid signal representing the occlusion parameter in the blood vessel, to convert the fluid signal to an electrical signal, and to communicate the electrical signal to the electronic control unit (22) via the first electrical communication interface (12) communicating with the second electric communication interface (13).

2. The device according to claim 1, comprising a storage body (23) for storage of the fluid medium.

3. The device according to claim 2, wherein the storage body is contained in the controller body (8).

4. The device according to any of the preceding claims, wherein the fluid signal is received by the electronic sensor converter via a sensor conduit (15) extending in the elongated member between an upstream location (18) and the electronic sensor converter (21).

5. The device according to claim 4, wherein the upstream location is between the distal end (4) and the inflatable member (6).

6. The device according to any of claims 4-5, comprising a purge structure (14) allowing filling of the sensor conduit (15) with a propagation medium.

7. The device according to claim 6, wherein the purge structure comprises an external access port (14) configured to connect a propagation medium container.

8. The device according to claims 6-7, wherein the purge structure comprises a confluence configured to establish fluid communication between the sensor conduit and the storage body to allow purging with the fluid medium in the storage body (23).

9. The device according to claim 8, wherein the confluence is configured to be controlled by at least one or more of the following: A pressure difference between pressure in the inflation conduit and pressure in the sensor conduit, such that it allows a fluid flow between the inflation conduit and the sensor conduit upon a pressure difference above a first threshold value and such that it prevents fluid flow between the inflation conduit and the sensor conduit upon a pressure difference below the first threshold value; An electronic valve, including an electronic solenoid valve, pinch valve or tube pinch valve.

10. The device according to any of the preceding claims, wherein the first electric communication interface and the first fluid communication interface are arranged to form a first mutual connection interface in the connector body, wherein the second electric communication interface and the second fluid communication interface are arranged to form a second mutual connection interface in the controller body, and wherein the first and second mutual connection interfaces are configured for establishing both electrical communication and fluid communication by joining the connector body and the controller body.

11. A method for preparing a device according to any of the preceding claims for use by connecting the connector body (7) to the controller body (8).

## Patentansprüche

1. Endovaskuläre Vorrichtung (1) zum Bewirken einer wenigstens teilweisen Okklusion in einem Blutgefäß in einem Individuum, wobei die Vorrichtung umfasst:
- einen länglichen Körper (2), der sich zwischen einem proximalen Ende (3) und einem distalen Ende (4) erstreckt, wobei das distale Ende in das Blutgefäß einführbar ist,
- ein aufblasbares Element (6), das um den länglichen Körper (2) herum ausgebildet ist und dafür ausgelegt ist, sich bei Aufnahme eines strömungsfähigen Mediums aus einem Aufblasmittel auszudehnen,
- einen Reglerkörper (8),
- einen Verbinderkörper (7), der an dem Reglerkörper (8) befestigbar ist und in fester Verbindung mit dem länglichen Körper (2) steht,
- ein erstes Schnittstellenmittel (9), das in dem Verbinderkörper (7) enthalten ist und einen externen Verbindungsanschluss bildet, der dafür ausgelegt ist, ein erstes manuell betätigtes Aufblasmittel anzuschließen,
- ein Abzweigelement (10), das in dem Verbinderkörper untergebracht ist und mit dem externen Verbindungsanschluss durch einen Fluiddurchgang verbunden ist,
- eine Aufblasleitung (16), die eine Fluidverbindung zwischen dem aufblasbaren Element und dem Abzweigelement (10) bereitstellt,
- eine erste Fluidverbindungsschnittstelle (11), die in dem Verbinderkörper (7) enthalten ist,
- eine erste elektrische Verbindungsschnittstelle (12), die in dem Verbinderkörper (7) enthalten ist,
- ein zweites kraftgesteuertes Aufblasmittel, das in dem Reglerkörper (8) angeordnet ist,
- eine zweite Fluidverbindungsschnittstelle (25), die in dem Reglerkörper (8) vorgesehen ist, wobei die erste Fluidverbindungsschnittstelle (11) und die zweite Fluidverbindungsschnittstelle (25) dafür ausgelegt sind, das zweite kraftgesteuerte Aufblasmittel anzuschließen, und mit dem Abzweigelement (10) in Fluidverbindung stehen,
- eine elektronische Steuereinheit (22), die dafür ausgelegt ist, ein elektrisches Signal zu empfangen, das einen Okklusionsparameter repräsentiert, und basierend auf dem Okklusionsparameter einen Anweisungssatz für manuell betätigtes Aufblasen oder für automatisch betätigtes Aufblasen bereitzustellen,
- eine zweite elektrische Verbindungsschnittstelle (13), die in dem in dem Reglerkörper (8) vorgesehen ist und dafür ausgelegt ist, mit der ersten elektrischen Verbindungsschnittstelle (12) elektrisch zu kommunizieren, und
- einen elektronischen Sensorwandler (21), der in dem Verbinderkörper (7) enthalten ist und dafür ausgelegt ist, ein Fluidsignal zu empfangen, das den Okklusionsparameter in dem Blutgefäß repräsentiert, das Fluidsignal in ein elektrisches Signal umzuwandeln und das elektrische Signal an die elektronische Steuereinheit (22) über die erste elektrische Verbindungsschnittstelle (12), die mit der zweiten elektrischen Verbindungsschnittstelle (13) kommuniziert, zu übermitteln.

2. Vorrichtung nach Anspruch 1, welche einen Speicherkörper (23) zur Speicherung des strömungsfähigen Mediums umfasst.

3. Vorrichtung nach Anspruch 2, wobei der Speicherkörper in dem Reglerkörper (8) enthalten ist.

4. Vorrichtung nach einem der vorhergehenden Ansprüche, wobei das Fluidsignal durch den elektronischen Sensorwandler über eine Sensorleitung (15) empfangen wird, die sich in dem länglichen Element zwischen einem stromaufwärtigen Ort (18) und dem elektronischen Sensorwandler (21) erstreckt.

5. Vorrichtung nach Anspruch 4, wobei sich der stromaufwärtige Ort zwischen dem distalen Ende (4) und dem aufblasbaren Element (6) befindet.

6. Vorrichtung nach einem der Ansprüche 4-5, welche eine Spülstruktur (14) umfasst, die ein Füllen der Sensorleitung (15) mit einem Ausbreitungsmedium ermöglicht.

7. Vorrichtung nach Anspruch 6, wobei die Spülstruktur einen Externzugangsanschluss (14) umfasst, der dafür ausgelegt ist, einen Ausbreitungsmediumbehälter anzuschließen.

8. Vorrichtung nach den Ansprüchen 6-7, wobei die Spülstruktur einen Zusammenfluss umfasst, der dafür ausgelegt ist, eine Fluidverbindung zwischen der Sensorleitung und dem Speicherkörper herzustellen, um ein Spülen mit dem strömungsfähigen Medium in dem Speicherkörper (23) zu ermöglichen.

9. Vorrichtung nach Anspruch 8, wobei der Zusammenfluss dafür ausgelegt ist, durch mindestens eines oder mehreres von Folgendem gesteuert zu werden: eine Druckdifferenz zwischen dem Druck in der Aufblasleitung und dem Druck in der Sensorleitung, derart, dass sie einen Fluidfluss zwischen der Aufblasleitung und der Sensorleitung bei einer Druckdifferenz oberhalb eines ersten Schwellenwertes ermöglicht, und derart, dass sie einen Fluidfluss zwischen der Aufblasleitung und der Sensorleitung bei einer Druckdifferenz unterhalb des ersten Schwellenwertes verhindert; ein elektronisches Ventil, wie etwa ein elektronisches Magnetventil, Quetschventil oder Rohrquetschventil.

10. Vorrichtung nach einem der vorhergehenden Ansprüche, wobei die erste elektrische Verbindungsschnittstelle und die erste Fluidverbindungsschnittstelle dazu eingerichtet sind, eine erste gegenseitige Verbindungsschnittstelle in dem Verbinderkörper zu bilden, wobei die zweite elektrische Verbindungsschnittstelle und die zweite Fluidverbindungsschnittstelle dazu eingerichtet sind, eine zweite gegenseitige Verbindungsschnittstelle in dem Reglerkörper zu bilden, und wobei die erste und die zweite gegenseitige Verbindungsschnittstelle dafür ausgelegt sind, durch Vereinigen des Verbinderkörpers und des Reglerkörpers sowohl eine elektrische Verbindung als auch eine Fluidverbindung herzustellen.

11. Verfahren zum Vorbereiten einer Vorrichtung nach einem der vorhergehenden Ansprüche für die Verwendung durch Anschließen des Verbinderkörpers (7) an den Reglerkörper (8).

## Revendications

1. Dispositif endovasculaire (1) pour fournir au moins une occlusion partielle dans un vaisseau sanguin dans un sujet, le dispositif comprenant :
- un corps allongé (2) s'étendant entre une extrémité proximale (3) et une extrémité distale (4), l'extrémité distale pouvant être insérée à l'intérieur du vaisseau sanguin,
- un membre gonflable (6) formé autour du corps allongé (2) et configuré pour entrer en expansion à la réception d'un agent fluidique venant d'un moyen de gonflage,
- un corps de commande (8),
- un corps de connecteur (7) pouvant être fixé au corps de commande (8) et en connexion fixe avec le corps allongé (2),
- un premier moyen d'interface (9) compris dans le corps de connecteur (7) et formant un port de connexion externe configuré pour la connexion à un premier moyen de gonflage manuel,
- une jonction (10) logée dans le corps de connecteur et connectée au port de connexion externe par un passage fluidique,
- un conduit de gonflage (16) fournissant une connexion fluidique entre le membre gonflable et la jonction (10),
- une première interface de communication fluidique (11) comprise dans le corps de connecteur (7),
- une première interface de communication électrique (12) comprise dans le corps de connecteur (7),
- un second moyen de gonflage à commande électrique situé dans le corps de commande (8),
- une seconde interface de communication fluidique (25) prévue dans le corps de commande (8), la première interface de communication fluidique (11) et la seconde interface de communication fluidique (25) étant configurées pour se connecter au second moyen de gonflage à commande électrique et étant en communication fluidique avec la jonction (10),
- une unité de commande électronique (22) configurée pour recevoir un signal électrique représentant un paramètre d'occlusion et pour fournir un jeu d'instructions pour un gonflage manuel ou pour un gonflage automatique en se basant sur le paramètre d'occlusion,
- une seconde interface de communication électrique (13) prévue dans le corps de commande (8) et configurée pour communiquer électriquement avec la première interface de communication électrique (12), et
- un convertisseur à capteur électronique (21) contenu dans le corps de connecteur (7) et configuré pour recevoir un signal fluidique représentant le paramètre d'occlusion dans le vaisseau sanguin, pour convertir le signal fluidique en un signal électrique, et pour communiquer le signal électrique à l'unité de commande électronique (22) par le biais de la première interface de communication électrique (12) communiquant avec la seconde interface de communication électrique (13).

2. Dispositif selon la revendication 1, comprenant un corps de stockage (23) pour stocker l'agent fluidique.

3. Dispositif selon la revendication 2, dans lequel le corps de stockage est contenu dans le corps de commande (8).

4. Dispositif selon l'une quelconque des revendications précédentes, dans lequel le signal fluidique est reçu par le convertisseur à capteur électronique par le biais d'un conduit de capteur (15) s'étendant dans le membre allongé entre un emplacement en amont (18) et le convertisseur à capteur électronique (21).

5. Dispositif selon la revendication 4, dans lequel l'emplacement en amont est entre l'extrémité distale (4) et le membre gonflable (6).

6. Dispositif selon l'une quelconque des revendications 4-5, comprenant une structure de purge (14) permettant le remplissage du conduit de capteur (15) avec un agent de propagation.

7. Dispositif selon la revendication 6, dans lequel la structure de purge comprend un port d'accès externe (14) configuré pour connecter un récipient d'agent de propagation.

8. Dispositif selon l'une des revendications 6-7, dans lequel la structure de purge comprend une confluence configurée pour établir une communication fluidique entre le conduit de capteur et le corps de stockage pour permettre la purge avec l'agent fluidique dans le corps de stockage (23).

9. Dispositif selon la revendication 8, dans lequel la confluence est configurée pour être commandée par au moins un ou plusieurs de ce qui suit : une différence de pression entre la pression dans le conduit d'inflation et la pression dans le conduit de capteur, de façon qu'elle permette un écoulement fluidique entre le conduit d'inflation et le conduit de capteur lors d'une différence de pression au-delà d'une première valeur seuil et de façon qu'elle empêche un écoulement fluidique entre le conduit d'inflation et le conduit de capteur lors une différence de pression en-deça de la première valeur seuil ; une soupape électronique, incluant une électrovanne, un robinet à manchon ou un robinet à manchon à tube.

10. Dispositif selon l'une quelconque des revendications précédentes, dans lequel la première interface de communication électrique et la première interface de communication fluidique sont agencées pour former une première interface de connexion mutuelle dans le corps de connecteur, dans lequel la seconde interface de connexion électrique et la seconde interface de communication fluidique sont agencées pour former une seconde interface de connexion mutuelle dans le corps de commande, et dans lequel les première et seconde interfaces de connexion mutuelle sont configurées pour établir à la fois la communication électrique et la communication fluidique en joignant le corps de connecteur et le corps de commande.

11. Procédé de préparation d'un dispositif selon l'une quelconque des revendications précédentes destiné à être utilisé par la connexion du corps de connecteur (7) au corps de commande (8).
